Europäisches Patentamt

European Patent Office

Office européen des brevets

(11) Publication number: **0 141 896**
**B1**

(12) **EUROPEAN PATENT SPECIFICATION**

(45) Date of publication of patent specification: 23.11.89

(21) Application number: 84104868.9

(22) Date of filing: 30.04.84

(51) Int. Cl.⁴: **A 61 F 5/37**

(54) Sleeved jacket restraining device.

(30) Priority: 16.09.83 US 533053

(43) Date of publication of application:
22.05.85 Bulletin 85/21

(45) Publication of the grant of the patent:
23.11.89 Bulletin 89/47

(84) Designated Contracting States:
DE FR GB

(56) References cited:
US-A-2 782 783
US-A-2 940 443
US-A-3 082 764
US-A-3 817 245
US-A-4 119 095
US-A-4 360 014

(73) Proprietor: **JOHN T. POSEY COMPANY**
**5635 Peck Road**
**Arcadia California 91006 (US)**

(72) Inventor: **Posey, John Thornton, Sr.**
**1739 Meadowbrook**
**Altadena California 91001 (US)**

(74) Representative: **Patentanwälte Grünecker,**
**Kinkeldey, Stockmair & Partner**
**Maximilianstrasse 58**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention refers to a restraining device for comfortably holding a patient in a hospital bed or wheelchair as set forth in the preamble of claim 1. Such restraining device is known from US—A—4 360 014.

In this document, a garment for comfortably and securely confining a patient in a bed, chair or other supporting structure is disclosed. The garment is a loose fitting gown disposed on the upper body that permits the patient mobility on the supporting structure yet prevents escape by accident or otherwise. The garment has waist and crotch restraining members which can be employed alternately and/or simultaneously for confining a patient to a bed, a chair or like-supporting structure. The garment having a size which is adapted to the size of the patient, a plurality of such garments of different sizes are needed in a hospital or like-institution in order to serve patients of different sizes. Furthermore, due to the relatively loose fit of the gown disposed on the upper body of the patient, the patient's mobility may be rather extensive so that the confinement provided by the garment is, in some cases, not sufficient. Furthermore, there are no means which are independent from the supporting structure and can prevent the patient from sliding down in the garment.

It is therefore, the object of the present invention to provided a restraining device as set forth in the preamble of claim 1 which may be applied to patients of different sizes and girth and which is adapted to securely restrain a patient in a hospital bed, wheelchair or a like-supporting structure.

This object is attained by the characterizing features of claim 1. Preferred embodiments of the invention are the subject of the dependent claims.

The invention, thus, provides a sleeved jacket restraining device having a combination of a jacket that fits over the shoulders and continuously around the midriff region of a patient, with sleeves extending along the arms of the patient to provide a further means of restraint. Pairs of circumferentially spaced-apart ties on the right and left sides of the jacket midriff region, where each tie is securely affixed to the jacket at one end, are provided, and the free ends of such ties may then be fastened to each other on both sides of the jacket to adjust the circumferential size of the jacket and to retain the adjusted size around the patient. Fastening means securely affixed to the front of the midriff region of the jacket and extending away from opposite sides of the jacket are provided from attachment to a fixture located remotely from the jacket worn by the patient.

Restraining means at the front and rear midriff region of the jacket are adapted for attachment to a restraining strap extending from the front restraining means under the patient between his legs into the rear restraining means of the jacket for preventing the patient from sliding down in the jacket and in the fixture to which said jacket is affixed.

In use, when the sleeved jacket is worn by a patient, such as a patient in a wheelchair, the right side ties and the left side ties are pulled toward one another and tied to provide adjustment for each side of the jacket so that the jacket is comfortable yet fits closely to the size and shape of the patient. The ties at the sides of the jacket can be fastened so that they cannot be loosened or untied by the patient, so that security is maintained as well as the comfort of the patient. The right and left portions of the restraining strap extend away from the front midriff region of the jacket and are attached to the under-structure of the wheelchair so that the patient is unable to free himself. The sleeve effectively restrains the patient in the wheelchair. The sleeves of the jacket combine with the ties at the sides and the waist strap at the bottom to restrain the patient from rising up, the strap extending between the legs and affixed to the jacket prevent the patient from sliding down or out of the jacket or pulling the jacket over his head. Although the patient is effectively restrained by the jacket, he remains comfortable. The jacket provides substantial freedom of movement for the patient without producing the annoying pressure on the upper torso and shoulders normally associated with tight shoulder straps or waist straps and the like which, when used alone, can produce discomforting pressure points that are avoided by the sleeved jacket of this invention. Nevertheless, by means of shoulder restraint means the freedom of movement of the patient may be confined, if necessary.

The invention will now be explained by means of a preferred embodiment shown in the drawings.

### Drawings

Fig. 1 is a perspective view showing the front side of a sleeved jacket restraining device, in which the rear of the jacket is open.

Fig. 2 is an elevation view showing the rear side of the restraining jacket, in which the jacket is closed.

Fig. 3 is a perspective view showing the sleeved jacket restraining device in use on a wheelchair patient.

Fig. 4 is a perspective view showing the sleeved jacket restraining device in use on a hospital bed patient.

### Detailed description

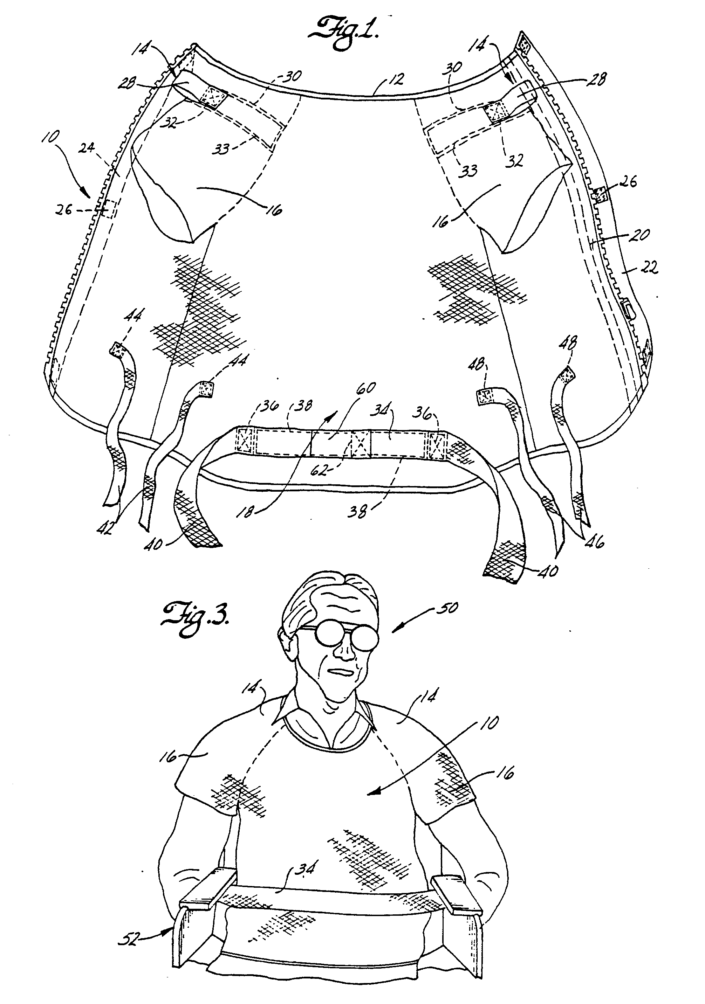

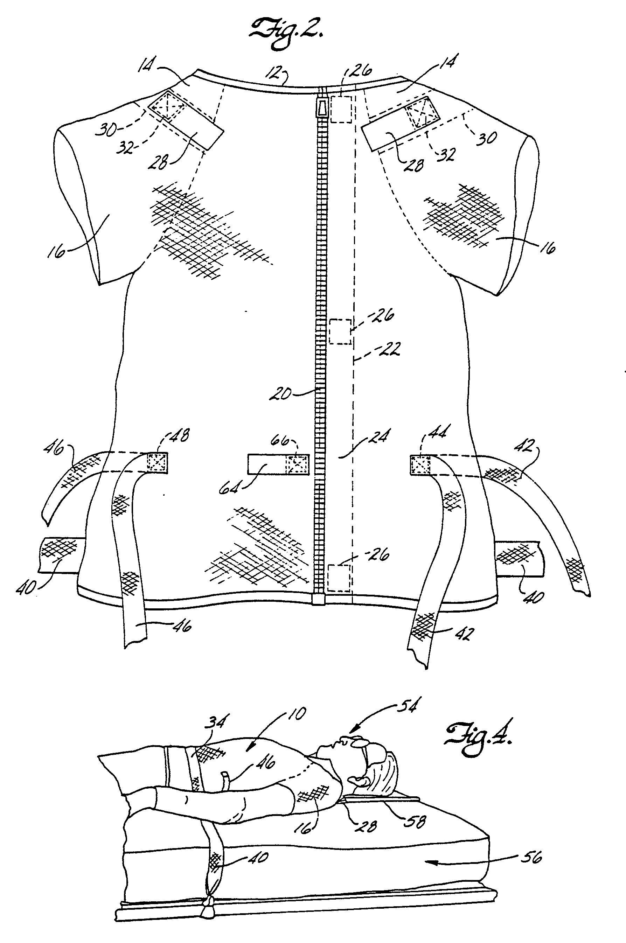

Referring to Figs. 1 and 2, a restraining device according to principles of this invention includes a sleeved jacket 10 having a top edge 12 for extending around the patient's neck when the jacket is worn, a pair of shoulder regions 14 extending over the patient's shoulders, and a pair of sleeves 16 at the sides of the jacket 10 for encircling the patient's arms. The sleeves 16 extend for about one-half the length of the patient's upper arms (to about the midpoint of the distance from the shoulders to the elbows). The jacket 10 extends downwardly a sufficient distance to define near its bottom a midriff section 18 adapted to be disposed around the midriff of the patient when the jacket 10 is worn. The jacket 10 opens and closes in the rear, and a zipper 20 is fastened to close the jacket 10

around the patient's upper torso. A narrow vertical margin 22 at the rear of the jacket 10 extends outboard of one portion of the zipper 20. A vertical margin 24 at the rear of the jacket 10 inboard of the other portion of the zipper 20 overlaps the narrow margin 22. Cooperating fasteners 26, preferrably burdock-like ones, are vertically spaced apart along the overlapping marginal portions 22 and 24. The fasteners 26 are attached to one another in the well known manner for closing the rear portion of the jacket 10. The zipper 20 can be fastened, if desired, to secure the jacket 10 in its closed position. As illustrated best in Fig. 2, the length of the zipper 20 is offset from the rear center of the jacket 10 so that the zipper 20, when closed, is not aligned with the patient's spinal column.

Left and right restraints in the form of loops 28 are fastened to the rear left and right shoulder regions of the jacket 10. Preferably, a separate elongated reinforcing strap 30 is fastened to the inside of the rear left and right shoulder regions of the jacket. Each loop 28 is fastened to a corresponding reinforcing strap 30 by stitching 32. The reinforcing straps 30 are fastened by stitching 33.

A long flexible strap 34 is secured to the front midriff section 18 of the jacket 10. Stitching 36 and 38 fastens the strap 34 across the front of the jacket 10, leaving long left and right flexible restraining end portions 40 extending freely away from the left and right sides of the jacket 10.

A pair of horizontally spaced apart right side ties 42 are secured to the right side of the jacket 10 by stitching 44. The ties 42 are flexible and extend freely away from the right lower side portion of the jacket 10. A similar pair of horizontally spaced apart left side ties 46 are secured by stitching 48 to corresponding portions of the left lower side of the jacket 10.

As shown in Fig. 1, a loop 60 can be affixed to the middle of the lower front of the jacket 10 by stitching 62. A cooperating loop 64 can be affixed to the middle of the lower back of the jacket 10 by stitching 66, as shown in Fig. 2.

In use, the sleeved jacket 10 secures a patient to a bed, wheelchair, or the like. Fig. 3 illustrates use of the jacket 10 for restraining a patient 50 sitting in a wheelchair 52. Fig. 4 illustrates use of the jacket 10 for restraining a patient 54 lying in a hospital bed 56. The jacket 10 is placed on the upper torso of the patient by extending the patient's arms through the sleeves 16 and then pulling the jacket 10 behind the patient. The fasteners 26 can be fastened before the zipper 20 is used to close the rear of the jacket 10. The right side ties 42 are then pulled toward one another and tied, and the left side ties 46 are also pulled toward one another and tied. The side ties 42, 46 provide adjustment for each side of the jacket 10 so that the jacket 10 is comfortable, yet fits closely to the size and shape of the patient's upper torso. The side ties 42, 46 are preferably tied so that they cannot be loosened or untied by the patient. The shoulder sections of the jacket 10 fit snuggly and comfortably around the patient's shoulders, and the sleeves 16 extend around the upper portions of the patient's arms. The jacket 10 extends downwardly so that its midriff section 18 is disposed snuggly yet comfortably around the midriff of the patient. Right and left end portions 40 of the restraining strap 34 are then extended away from the sides of the patient for attachment to the understructure of the wheelchair or the bed at locations remote from the patient so that he is unable to free himself. The restraining loops 28 also can be used to provide additional restraint. For a wheelchair patient, the loops may be attached to the rear handles of the wheelchair, or a strap (not shown) may be threaded through the loops and attached to the handles of the wheelchair. For a patient in a hospital bed, a strap 58 can be threaded through the loops 28 and attached to remote locations of the bed's understructure. When the jacket is in place, the zipper 20 is offset from the patient's spinal column so that added pressure against the spinal column is not produced by the zipper 20 as the patient either sits in the wheelchair or lies in the hospital bed. A crotch strap (not shown) can be placed between the patient's legs and connecting the two loops 60 and 64 shown in Figs. 1 and 2. Thus can add further safety in preventing the patient from sliding down in the jacet 10.

The sleeved jacket effectively restrains the patient in the wheelchair or bed. The sleeves of the jacket combined with the ties at the sides and the waist straps at the bottom restrain the patient from raising up, sliding down out of the jacket, or pulling the jacket over his head. Although the patient is effectively restrained by the jacket, he remains comfortable. The jacket provides substantial freedom of movement for the patient's upper torso, his arms and shoulders. This movement is possible without producing the annoying pressure on the upper torso and shoulders normally associated with tight shoulder straps, or the like. Since the waist straps are not wrapped around the midriff of the patient, a substantial amount of lateral movement is allowed, which avoids discomforting pressure around the waist normally produced by conventional waist belts and the like.

The jacket is preferably made from a strong flexible material such as cotton fabric, although the jacket also can be made from nylon. The attendant waist straps and loops are preferably made from a strong flexible material such as nylon.

**Claims** —

1. A restraining device for comfortably holding a patient in a hospital bed or wheelchair, the restraining device comprising:

a jacket (10) adapted to be disposed around the upper torso of the patient so that it makes a snug fit around the shoulders of the patient and extends downwardly around the midriff of the patient, the jacket (10) having sleeves (16) for extending along the arms of the patient;

adjustment means (42, 46) on both sides of the midriff region (18) of the jacket (10) for adjusting the size of the jacket midriff region (18) to the size of the patient; and

fastening means (40) in the midriff region (18) of the jacket (10) extending away from opposite sides of the jacket (10) for attachment to a fixture, characterized in that said adjustment means (42, 46) are designed for retaining the adjusted size of the jacket (10) and comprise a pair of right side ties (42) spaced apart circumferentially from one another on the right side of the midriff region (18) of the jacket (10), and a pair of left side ties (46) spaced apart circumferentially from one another on the left side of the midriff region (18) of the jacket (10), each pair of spaced apart ties (42, 46) having inner ends (44, 48) securely affixed to the jacket midriff region (18) and free ends spaced from the jacket (10) so that the ties (42, 46) on each side of the jacket (10) can be fastened to one another for adjusting the circumferential size of the jacket midriff region (18) to fit the size of the patient and for retaining the adjusted size of the jacket (10), that the fastening means (40) are securely affixed to the front of the midriff region (18) of the jacket (10) and that front and rear restraining means (60, 64) are attached to the front and rear of the jacket (10), respectively, for attachment to front and rear portions of a strap to extend between the legs of the patient from the front restraining means (60) to the rear restraining means (64) to prevent the patient from sliding down in the jacket (10).

2. Restraining device according to claim 1 in which the jacket (10) opens and closes in the rear, and a zipper (20) is fastened to close the jacket (10) around the patient's upper torso said zipper (20) being offset from the rear center of the jacket (10) so as, when closed, to be offset from the patient's spinal column.

3. Restraining device according to claim 2 including a narrow vertical margin (22) at the rear of the jacket (10) extending outboard of one portion of the zipper (20) and another narrow vertical margin (24) at the rear of the jacket (10) inboard of the other portion of the zipper (20), said margins (22, 24) overlapping each other in closed condition of said zipper (20).

4. Restraining device according to claim 3 including fastening means (26) for fastening the margins (22, 24) when overlapping each other.

5. Restraining device according to claims 1 or 2, including restraining means (28) on the rear shoulder regions (14) of the jacket (10) for use in attachment to the fixture.

6. Restraining device according to any one of claims 1 to 3, in which the fastening means on the midriff region (18) of the jacket (10) comprises an elongated waist strap (34) having a central region securely affixed to the midriff region (18) of the jacket (10) with the opposite right and left flexible free end portions extending away from opposite sides of the jacket (10), for attachment remotely to the fixture.

7. Restraining device as set forth in any one of the preceding claims, the jacket (10) having sleeves (16) for extending along the arms of the patient.

8. A restraining device according to any one of the preceding claims including shoulder restraining means (28) on the rear shoulder regions of the jacket (10) for use in attachment to the fixture.

**Patentansprüche**

1. Festhaltevorrichtung zum bequemen Halten eines Patienten in einem Krankenhausbett oder einem Rollstuhl, enthaltend:

eine Weste (10), die dazu eingerichtet ist, um den Oberkörper des Patienten agneordnet zu werden, so daß sie um die Schultern des Patienten eng sitzt und sich un die Taille des Patienten nach unten erstreckt, welche Weste (10) Ärmel (16) zur Erstreckung längs der Arme des Patienten aufweist;

Einstelleinrichtungen (42, 46) auf beiden Seiten des Taillenbereiches (18) der Weste (10) zum Anpassen der Größe des Taillenbereichs (18) der Weste an die Größe des Patienten; und

Befestigungseinrichtungen (40) im Taillenbereich (18) der Weste (10), die sich von gegenüberliegenden Seiten der Weste (10) zur Anbringung an einer Halteeinrichtung erstrecken,

dadurch gekennzeichnet, daß die Einstelleinrichtungen (42, 46) dazu eingerichtet sind, die eingestellte Größe der Weste (10) aufrechtzuerhalten, und daß sie enthalten: ein Paar rechte Bänder (42), die in Umfangsrichtung voneinander entfernt auf der rechten Seite des Taillenbereiches (18) der Weste (10) angeordnet sind, und ein Paar linke Bänder (46), die im Umfangsrichtung voneinander beabstandet auf der linken Seite des Taillenbereiches (18) der Weste (10) angeordnet sind, wobei jedes Paar beabstandeter Bänder (42, 46) innere Enden (44, 48) aufweist, die fest an dem Taillenbereich (18) der Weste befestigt sind, und freie Enden aufweist, die von der Weste (10) Abstand haben, so daß die Bänder (42, 46) auf jeder Seite der Weste (10) aneinander befestigt werden können, um die Umfangsgröße des Taillenbereiches (18) der Weste zu verstellen, um sie an die Größe des Patienten anzupassen und um die eingestellte Größe der Weste aufrechtzuerhalten, daß die Befestigungseinrichtungen (40) fest an der Vorderseite des Taillenbereiches (18) der Weste angebracht sind und daß vordere und hintere Festhalteeinrichtungen (60, 64) an der Vorderseite bzw. der Rückseite der Weste (10) befestigt sind zur Befestigung der vorderen und hinteren Abschnitte eines Bandes zur Erstreckung zwischen den Füßen des Patienten von der vorderen Festhalteeinrichtung (60) zur hinteren Festhalteeinrichtung (64), um zu verhindern, daß der Patient in der Weste (10) nach unter rutscht.

2. Festhaltevorrichtung nach Anspruch 1, bei der die Weste (10) sich an der Rückseite öffnet und schließt und ein Reißverschluß (20) angebracht ist, um die Weste (10) um den Oberkörper des Patienten zum Schließen, welcher Reißverschluß (20) gegenüber der rückwärtigen Mitte der Weste (10) versetzt ist, um im geschlossenen Zustand gegenüber der Wirbelsäule des Patienten versetzt zu sein.

3. Festhaltevorrichtung nach Anspruch 2, enthaltend einen schmalen vertikalen Randstreifen (22) an der Rückseite der Weste (10), der sich vom einen Teil des Reißverschlusses (20) nach außen erstreckt, und einen weiteren schmalen vertikalen Randstreifen (24), der sich an der Rückseite der Weste (10) von dem anderen Teil des Reißverschlusses (20) nach innen erstreckt, welche Randstreifen (22, 24) einander im geschlossenen Zustand des Reißverschlusses (20) überlappen.

4. Festhaltevorrichtung nach Anspruch 3, enthaltend Befestigungseinrichtungen (26) zum Befestigen der Randstreifen (22, 24), wenn sie einander überlappen.

5. Festhaltevorrichtung nach den Ansprüchen 1 oder 2, enthaltend Festhalteeinrichtungen (28) an den hinteren Schulterbereichen (14) der Weste zur Verwendung bei der Anbringung an der Halteeinrichtung.

6. Festhaltevorrichtung nach einem der Ansprüche 1 bis 3, bei der die Befestigungseinrichtung am Taillenbereich (18) der Weste (10) ein langgestrecktes Bauchband (34) enthält, das einen Mittenabschnitt aufweist, der am Taillenbereich (18) der Weste fest angebracht ist, wobei die entgegengesetzten rechten und linken flexiblen freien Endabschnitte sich von entgegengesetzten Seiten der Weste (10) zur entfernten Befestigung an der Halteeinrichtung erstrecken.

7. Festhaltevorrichtung nach einem der vorhergehenden Ansprüche, bei der die Weste (10) Ärmel (16) zur Erstreckungs längs der Arme des Patienten aufweist.

8. Festhaltevorrichtung nach einem der vorhergehenden Ansprüche, enthaltend Schulterfesthalteeinrichtungen (28) an den hinteren Schulterabschnitten der Weste (10) zur Verwendung bei der Anbringung an der Halteeinrichtung.

**Revendications**

1. Dispositif de retenue pour maintenir confortablement un patient dans un lit d'hôpital ou un fauteuil roulant, le dispositif de maintien comprenant:

un gilet (10) adapté pour être placé autour du haut du torse du patient de sorte qu'il est bien ajusté autour des épaules du patient et s'étend vers le bas autour de la taille du patient, le gilet (10) ayant des manches (16) qui s'étendent le long des bras du patient;

des moyens d'ajustage (42, 46) sur les deux côtés de la région de taille (18) du gilet (10) pour ajuster la taille de la région de taille (18) du gilet à la taille du patient; et

des moyens d'attache (40) dans la région de taille (18) du gilet (10) s'étendant en sens opposé aux côtés opposés du gilet (10) pour être attachés à une fixation, caractérisé en ce que lesdits moyens d'ajustage (42, 46) sont conçus pour maintenir la taille ajustée du gilet (10) et comprennent une paire de cordons latéraux droits (42) espacés circonférentiellement l'un de l'autre sur le côté droit de la région de taille (18) du gilet (10), et une paire de cordons latéraux gauches (46) espacés circonférentiellement l'un de l'autre sur le côté gauche de la région de taille (18) du gilet (10), chaque paire de cordons espacés (42, 46) ayant des extrémités intérieures (44, 48) fixées solidement à la région de taille (18) du gilet et des extrémités libres espacées du gilet (10) de sorte que les cordons (42, 46) sur chaque côte du gilet (10) peuvent être attachés l'un à l'autre pour ajuster la taille circonférentielle de la région de taille (18) du gilet pour s'ajuster à la taille du patient et pour maintenir la taille ajustée du gilet (10), en ce que les moyens d'attache (40) sont fixés solidement au devant de la région de taille (18) du gilet (10) et en ce que des moyens de retenue avant et arrière (60, 64) sont attachés à l'avant et à l'arrière du gilet (10), respectivement, pour être fixés aux parties avant et arrière d'une sangle pour s'étendre entre les jambes du patient depuis les moyens de retenue avant (60) aux moyens de maintien arrière (64) afin d'empêcher le patient de des cendre en glissant dans le gilet (10).

2. Dispositif de retenue selon la revendication 1, dans lequel le gilet (10) s'ouvre et se ferme à l'arrière, et une fermeture éclair (20) est attachée pour fermer le gilet (10) autour du haut du torse du patient, ladite fermeture éclair (20) étant décalée par rapport au milieu arrière du gilet (10) de manière, lorsqu'elle est fermée, à être décalée par rapport à la colonne vertébrale du patient.

3. Dispositif de retenue selon la revendication 2, comprenant une lisière verticale étroite (22) à l'arrière du gilet (10) qui s'étend en dehors d'une première partie de la fermeture éclair (20) et une autre lisière verticale étroite (24) à l'arrière du gilet (10) à l'intérieur de l'autre partie de la fermeture éclair (20), lesdites lisières (22, 24) se recouvrant l'une l'autre à l'état fermé de ladite fermeture éclair (20).

4. Dispositif de retenue selon la revendication 3, comprenant des moyens d'attache (26) pour attacher les lisières (22, 24) lorsqu'elles se recouvrent l'une l'autre.

5. Dispositif de retenue selon les revendications 1 ou 2, comprenant des moyens de retenue (28) sur les régions d'épaules arrière (14) du gilet (10) utilisés pour être attachés à la fixation.

6. Dispositif de retenue selon l'une quelconque des revendications 1 à 3, dans lequel les moyens d'attache sur la région de taille (18) du gilet (10) comprennent une sangle de ceinture allongée (34) ayant une région centrale fixée solidement à la région de taille (18) du gilet (10), les parties terminales libres flexibles, opposées, gauche et droite, s'étendant en sens opposé aux côtes opposés du gilet (10), pour être attachées de façon éloignée à la fixation.

7. Dispositif de retenue comme indiqué dans l'une quelconque des précédentes revendications, le gilet (10) ayant des manches (16) pour s'étendre le long des bras du patient.

EP 0 141 896 B1

8. Dispositif de retenue selon l'une quelconque des précédentes revendications, comprenant des moyens de retenue d'épaules (28) sur les régions d'épaule arrière du gilet (10) pour être attachés à la fixation.

*Fig.1.*

*Fig.3.*

EP 0 141 896 B1

Fig.2.

Fig.4.

2